# EUROPÄISCHE PATENTSCHRIFT

(11) **EP 3 476 432 B1**
(45) Veröffentlichungstag und Bekanntmachung des Hinweises auf die Patenterteilung: **16.12.2020**
(21) Anmeldenummer: 18200923.3
(22) Anmeldetag: 17.10.2018
(51) Int. Cl.: A61N 1/39, A61B 5/0245

(54) **VORRICHTUNG ZUR HOCHSPANNUNGSTHERAPIE**
DEVICE FOR HIGH VOLTAGE TREATMENT
DISPOSITIF DE THÉRAPIE DE HAUTE TENSION

(30) Priorität: 26.10.2017 DE 102017125044
(43) Veröffentlichungstag der Anmeldung: 01.05.2019
(73) Patentinhaber: BIOTRONIK SE & Co. KG, 12359 Berlin (DE)
(72) Erfinder: Weiss, Ingo, 12435 Berlin (DE); Friedrich, Michael, 14532 Kleinmachnow (DE); Dörr, Thomas, 12437 Berlin (DE); Feese, Ulrich, 14167 Berlin (DE); Schlodder, Karsten, 15517 Fürstenwalde (DE); Kolberg, Gernot, 12161 Berlin (DE)
(74) Vertreter: Keck, Hans-Georg

(56) Entgegenhaltungen:
- WO-A1-2007/068284
- WO-A1-2016/149623
- WO-A2-2006/061725
- WO-A2-2012/150000
- US-A1- 2012 150 247

## Beschreibung

Gegenstand der Erfindung sind Vorrichtungen und Systeme zur elektrischen Stimulationstherapie an einem Patienten. Insbesondere betrifft die Erfindung das Gebiet der Herzstimulation, in einem konkreten Beispiel implantierbare Systeme zur Kardioversion und Defibrillation des Herzens.

Bekannt sind implantierbare Systeme zur Herzstimulation, Kardioversion und Defibrillation ICDs (implantable Cardioverter-Defibrillator) bestehend aus einem Implantatgehäuse, beinhaltend Energieversorgung, Kondensatoren, Elektronikmodule etc., und einem oder mehreren Elektrodenleitungen. Die Elektrodenleitungen verfügen über einen oder mehreren Elektroden zur Messung von Herzpotentialen und/oder Abgabe von Stimulationspulsen. Elektrodenleitungen mit einer Schock-Elektrode (Schock-Spule) sind in der Lage, einen intrakardialen Defibrillationsschock im Falle von lebensgefährlichen kardialen Arrhythmien abzugeben. Die Elektrodenleitungen sind mit dem Implantatgehäuse verbunden über in ein Headermodul integrierte Konnektoranschlüsse.

Bekannt aus dem Stand der Technik sind ebenfalls Elektrotherapiesysteme zur Stimulation, Kardioversion und Defibrillation des Herzens, bei denen die Kardioversion- und Schockfunktion ausgelagert ist auf ein subkutanes Implantat, einen sogenannten sICD (subcutaneous Implantable Cardioverter-Defibrillator), welches eine extravaskuläre Defibrillation durchführt. Der sICD wird unter die Haut des Patienten implantiert, wobei mindestens zwei Elektrodenpole für die Abgabe des Schocks derart angeordnet sind, sodass der Strompfad ("Schock-Vektor") durch die zu schockenden Areale des Herzens führt. Bekannt sind sICDs bestehend aus einem Implantatgehäuse und konnektierter Schockelektrodenleitung, wobei das Gehäuse seitlich über den Rippen in Achselnähe, und die Schockelektrodenleitung mittig über der Brust implantiert wird.

Vorteile eines sICD Systems im Vergleich zu einem ICD System bestehen darin, dass keine intrakardiale Elektrode platziert werden muss, sodass das Risiko für den Patienten sinkt. Nachteile eines sICD Systems bestehen in der höheren Schockenergie, die durch den längeren Stromweg von den Schockelektroden bis zum Herzgewebe erforderlich sind. So erfordern bekannte sICD Systeme hohe Therapiespannungen größer 1400 Volt und Energien größer 80 Joule pro Schock. Anforderungen an die Hardware solcher sICD Systeme sind somit entsprechend höher als jener von herkömmlichen ICDs. Es werden spezielle, teure Hochspannungsbauelemente (Ladeschaltung, Hochspannungs-Kondensatoren), großräumige Layouts zur Einhaltung der Durchschlagstrecken etc. benötigt. Durch die spezielleren Hardware-Komponenten steigt das Volumen des Implantatgehäuses des sICDs, sodass das Gerät ist deutlich größer ist als herkömmliche ICDs und damit wesentlich störender für den Patienten.

WO 2016/149623 A1 offenbart ein implantierbares medizinisches Gerät, das über mehrere Energiequellen verfügt, wobei die Energiequellen kombiniert werden können zur Erzeugung einer Stimulationsspannung. Es wird außerdem ein System aus mehreren implantierbaren medizinischen Geräten ("Subsystems"), beschrieben, welche jeweils über mindestens eine Energiequelle verfügen. Das implantierbare medizinische Gerät oder Gerätesystem ist dazu ausgelegt, eine Stimulationsspannung zu generieren, in dem die mehreren Energiequellen kombiniert werden. Dabei soll jeweils eine bestimmte Phase der Stimulationsspannung aus einer Energiequelle erzeugt werden. Die finale Stimulationsspannung wird aus den verschiedenen Phasen zusammengesetzt. Hardwaretechnisch wird das Zusammensetzen der einzelnen Phasen zu einer Stimulationsspannung durch schnelles Schalten ermöglicht. Die Schalter können Teil einer Kontrolleinheit oder des Pulsgenerators sein.

Es ist deshalb Aufgabe der vorliegenden Erfindung, ein Therapiesystem zu entwickeln, das die oben genannten Nachteile nicht aufweist und verglichen mit bekannten Lösungen leichter und kostengünstiger realisierbar ist. Die Erfindung soll das Problem lösen, dass Therapiegeräte (implantierbar oder nicht implantierbar) in der Elektrotherapie oft großvolumig ausgelegt werden müssen und die Verwendung von teuren Spezialbauelementen erfordern, um die benötigten Energien und Spannungen für die Therapie zu erzeugen. Insbesondere soll die erfindungsgemäße Lösung für die extravaskuläre Defibrillation eine effektive Therapiespannung sicherstellen, ohne dass spezielle, hochvolumige Hochspannungsbauelemente erforderlich sind.

### Beschreibung der Erfindung

Die Aufgabe wird erfindungsgemäß durch die Merkmale des unabhängigen Anspruchs 1 gelöst. Günstige Ausgestaltungen und Vorteile der Erfindung ergeben sich aus den weiteren Ansprüchen und der Beschreibung.

Gemäß einem ersten Aspekt der Erfindung wird eine Anordnung zur elektrischen Therapie für einen Patienten beschrieben, wobei die Anordnung dazu ausgelegt ist, zumindest eine elektrische Therapiespannung zu erzeugen, die an einem Therapieort im oder am Körper des Patienten wirkt. Die Anordnung umfasst mindestens eine erste und eine zweite Untereinheit, und jede Untereinheit weist zumindest einen Energiespeicher auf. Jede von einem einzelnen Energiespeicher speicherbare Energiemenge ist geringer als die zur Erzeugung der elektrischen Therapiespannung erforderliche Energiemenge. Jede Untereinheit weist zumindest eine der folgenden Komponenten auf: Detektionseinheit zur Detektion von Körperparametem, Therapieeinheit zur Erzeugung und/oder Abgabe einer elektrischen Spannungzur elektrischen Therapie, Steuereinheit, und/oder Kommunikationseinheit. Die Anordnung ist dazu ausgelegt, dass eine Untereinheit bezüglich der Erzeugung und/oder Abgabe einer elektrischen Spannung eine Master-Rolle einnimmt, und die zumindest eine restliche Untereinheit eine Slave-Rolle einnimmt.

Gegenstand der Erfindung ist eine Anordnung zur elektrischen Therapie für einen Patienten, wobei die Anordnung mindestens zwei Untereinheiten aufweist. Die Untereinheiten stellen jeweils eine Spannung für die elektrische Therapie bereit, wobei jede von einer Untereinheit bereitgestellte Spannung geringer ist, als die an einem Therapieort erreichte therapeutisch wirksame Spannung.

Jede Untereinheit weist zumindest eine der folgenden Komponenten auf:
Detektionseinheit zur Detektion von Körperparametern,

Therapieeinheit zur Erzeugung und/oder Abgabe einer elektrischen Spannung zur elektrischen Therapie, Steuereinheit, und/oder Kommunikationseinheit.

Eine Untereinheit nimmt bezüglich der Erzeugung und/oder Abgabe einer elektrischen Spannung eine Master-Rolle ein. Die zumindest eine restliche Untereinheit übernimmt eine Slave-Rolle, sodass die zumindest eine sich in der Slave-Rolle befindliche Untereinheit bezüglich des Zeitpunkts der Therapieabgabe und/oder Polarität der Therapieabgabe und/oder Dauer der Therapieabgabe an der Untereinheit in der Master-Rolle orientiert

Gemäß einem Aspekt der erfinderischen Lösung wird die erforderliche Therapiespannung durch Überlagerung von Teilbeiträgen aus mehreren Untereinheiten der Anordnung erzeugt.

Gemäß einer bevorzugten Ausführungsform der erfindungsgemäßen Anordnung sind die mindestens erste und zweite Untereinheit jeweils dazu ausgelegt, über Energie aus dem Energiespeicher eine elektrische Spannung zu erzeugen. Dabei ist die Anordnung dazu ausgelegt, die elektrische Therapiespannung zu erzeugen, in dem die von der zumindest ersten und zweiten Untereinheit erzeugten Spannungen überlagert werden.

In einer Ausführungsform der vorliegenden Erfindung werden die von den zumindest zwei Untereinheiten erzeugten Spannungen so überlagert, dass die Amplitude der überlagerten Spannung höher ist als die Amplitude einer durch eine Untereinheit erzeugten Spannung.

In einer Ausführungsform der vorliegenden Erfindung wobei die erste Untereinheit in einem ersten Außengehäuse angeordnet ist, und die zweite Untereinheit in einem zweiten Außengehäuse angeordnet ist. Beispielsweise handelt es sich bei der zumindest ersten und zweiten Untereinheit um einzelne Komponenten eines Therapiesystems, wie Batterie und Kondensator. Werden diese hochvolumigen Komponenten in getrennten Gehäusen untergebracht und untereinander verschaltet, wird das Gerätevolumen eines solchen Therapiegeräts auf zwei einzelne Geräte verteilt. Es kann sich bei den mindestens zwei Untereinheiten auch um einzelne Therapiegeräte handeln, wie zum Beispiel um subkutane implantierbare Kardioverter-Defibrillatoren (subcutaneous Implantable Cardioverter-Defibrillator, sICDs), oder um ein Therapiegerät und ein Gerät zur Messung von physiologischen Signalen ohne Therapiefunktion, wie z.B. im Bereich der Herzimplantate ein Loop-Recorder zur dauerhaften Aufnahme von Herzsignalen.

Gemäß einer weiteren Ausführungsform der vorliegenden Erfindung sind die zumindest erste und zweite Untereinheit in einem gemeinsamen Außengehäuse angeordnet. Denkbar ist beispielsweise die Anordnung von zwei Therapiegeräten mit Möglichkeit der Erzeugung einer Spannung zur Elektrostimulation in einem Gehäuse. Zur Erzeugung einer Therapiespannung werden die jeweiligen Spannungen so abgegeben, dass sie am Therapieort sich konstruktiv / im Wesentlichen phasensynchron überlagern, sodass eine hohe Amplitude der Therapiespannung entsteht. Jedes einzelne Therapiegerät kann dabei bezüglich des Volumens kleiner ausgelegt sein als wenn ein einziges Therapiegerät entworfen würde, sodass Reduktion des Gesamtvolumens und Freiheiten in der Gestaltung von Hardwarearchitektur und -Anordnung gegeben sind.

Gemäß einer bevorzugten Ausführungsform der vorliegenden Erfindung werden die von den zumindest zwei Untereinheiten erzeugten Spannungen so überlagert, dass die Spannungen am Therapieort im Wesentlichen phasensynchron sind. Im Wesentlichen phasensynchron ist im Kontext der Erfindung so zu verstehen, dass es sich bei den Spannungen um elektrische Wechselspannungen handelt, die am Therapieort so überlagert werden, dass die Amplitude der überlagerten Spannung (was die elektrische Therapiespannung am Therapieort darstellt) höher ist als jede Amplitude der einzelnen Wechselspannungen. Vorzugsweise besitzen die Wechselspannungen dieselben Frequenzen und eine Phasenverschiebung von 0°+/- 2π. Kleine Toleranzen in Phasenverschiebung und Frequenz sind nicht vermeidbar und mit umfasst.

Gemäß einer Ausführungsform der vorliegenden Erfindung ist jede Untereinheit dazu ausgelegt, zumindest mit einer anderen Untereinheit zu kommunizieren, wobei die Kommunikation galvanisch, elektromagnetisch, optisch, mechanisch und/oder akustisch stattfindet. Die Untereinheit kann dazu einen entsprechenden Transmitter/Receiver aufweisen.

In einer bevorzugten Ausführungsform der Erfindung weist mindestens eine Untereinheit eine Steuereinheit auf, wobei die Steuereinheit dazu ausgeführt ist, einen Therapiebefehl auszugeben, wobei der Therapiebefehl Informationen über zumindest einen der folgenden Parameter trägt:
Angaben zu Phase der jeweiligen von der Untereinheit bereitgestellten Spannung,
Zeitpunkt der Therapieabgabe,
Polarität der Therapieabgabe, und/oder
Dauer der Therapieabgabe bezogen auf die jeweilige Untereinheit.

Vorzugsweise erhält jene Untereinheit eine Master-Rolle erhält, die einen therapieerfordernden Körperparameter zuerst, d.h. vor allen anderen Untereinheiten, detektiert.

In einem Beispiel detektieren alle Untereinheiten ein physiologisches Signal. Sobald eine erste Untereinheit ein physiologisches Event misst, das eine Therapie erfordert, erzeugt die Untereinheit eine Spannung und gibt die Spannung an den Therapieort ab. Sobald die zumindest eine restliche Untereinheit die Spannungsabgabe der ersten Untereinheit detektiert, beginnt auch diese Untereinheit mit der Spannungserzeugung und -abgabe.

Gemäß einem weiteren Aspekt der Erfindung weist die Anordnung mindestens eine Verbindungsanordnung auf, wobei die mindestens zwei Untereinheiten durch die Verbindungsanordnung elektrisch verbunden sind, und wobei die Verbindungsanordnung vom Körper des Patienten elektrisch isoliert ist. Dabei können die mindestens zwei Untereinheiten über die Verbindungsanordnung seriell, antiseriell, parallel oder sternförmig verbunden sein.

Gemäß einer Ausführungsform der Erfindung wird die wesentliche Phasensynchronität der von den mindestens zwei Untereinheiten erzeugten Spannungen am Therapieort von der Anordnung über die mindestens eine Verbindungsanordnung erzeugt.

In weiteren erfindungsgemäßen Ausführungsformen der Erfindung umfassen die zumindest zwei Untereinheiten
- jeweils eine Batterie umfasst, und/oder
- jeweils einen Kondensator, und/oder
- es handelt sich bei den Untereinheiten jeweils um einen subkutanen implantierbaren Kardioverter-Defibrillator (sICD).

Entsprechend der Erfindung kann durch die räumliche Trennung verschiedener, für die Erzeugung einer Therapiespannung erforderlicher Komponenten auf verschiedene Untereinheiten auf ein einziges hochvolumiges Gerät verzichtet werden. Auch umfasst die Erfindung die Idee, einzelne kleinvolumigere Therapiegeräte im Zusammenwirken eine hohe Therapiespannung erzeugen zu lassen. Diese kleineren Therapiegeräte können jeweils ein eigenes Außengehäuse besitzen, oder in einem gemeinsamen Außengehäuse angeordnet sein. Die Erfindung sieht vor, dass mehr als ein Gerät derart konfiguriert sind, dass sie in koordinierter Operation eine Therapiespannung am Therapieort erzeugen können. Die Therapiespannung kann dabei dadurch erzeugt werden, dass Spannungen, die durch einzelne Untereinheiten erzeugt werden, am Therapieort überlagern bzw. sich addieren, sodass die erforderliche Therapieenergie/Therapiespannung appliziert werden kann.

In einer bevorzugten Ausführung der Anordnung ist zumindest eine Untereinheit dazu ausgelegt, ein Elektrokardiogramm oder ein subkutanes Elektrokardiogramm aufzunehmen.

In einer vorteilhaften Ausführung der Anordnung handelt es sich bei den Untereinheiten um sICDs. Durch eine Verschaltung zweier oder mehrerer sICDs wird erreicht, sodass sich die Spannungen am Therapieort aufsummieren. In einer Ausführung der erfindungsgemäßen Anordnung sind die Untereinheiten antiseriell verschaltet. Gemäß eines Ausführungsbeispiels der Erfindung gehören die Untereinheiten jeweils einer Spannungsklasse unter 1000 Volt an. So wird über die erfindungsgemäße Anordnung durch Überlagerung der Spannungen der Untereinheiten die Erzeugung einer Therapiespannung ermöglicht, die über der Spannung einer Untereinheit liegt (hier über 1000 Volt, z.B. für die extravenöse Defibrillation). Auf diese Weise kann mit kosteneffizienter Gerätetechnik der Spannungsklasse kleiner 1000V eine Therapiespannung generiert werden, die über 1000 Volt liegt.

Gemäß eines weiteren Aspekts der vorliegenden Erfindung sollten die Spannungen der Untereinheiten phasensynchron überlagert werden. Auf diese Weise wird eine gezielte Generierung einer effektiven Therapiespannung ermöglicht. Um die phasensynchrone Überlagerung der Spannungen zu gewährleisten, werden die beteiligten Untereinheiten synchronisiert. In einer Ausführungsform der erfindungsgemäßen Anordnung erfolgt die Synchronisation der Untereinheiten durch galvanische, optische, elektromagnetische oder mechanisch/akustische Kommunikation zwischen den Untereinheiten.

Gegenstand der Erfindung ist eine im Körper verteilt implantierte Vorrichtung zur Hochspannungstherapie dadurch gekennzeichnet, dass die energiespeichernden Elemente in örtlich getrennten Untereinheiten untergebracht sind, die elektrisch so miteinander verbunden sind, dass die therapeutische Wirkung durch synchronisierte Überlagerung von Teilbeiträgen aus den einzelnen Untereinheiten realisiert wird. Die Synchronisation erfolgt über galvanische, elektromagnetische, optische, mechanische oder akustische Kommunikation zwischen den Untereinheiten.

In einer Ausführungsform der Erfindung verfügt die Anordnung über zumindest ein Verbindungselement, über das die Untereinheiten elektrisch verbunden sind. Das Verbindungselement ist zum Körper des Patienten elektrisch isoliert.

In einer weiteren Ausführungsform verfügen die Untereinheiten über mindestens einen Anschluss, um mindestens eine weitere Untereinheit über eine Verbindungsleitung zu verbinden.

Jede Untereinheit verfügt über mindestens ein energiespeicherndes Element. Die energiespeichernden Elemente sind beispielsweise Batterien (Primärzellen und/oder Sekundärzellen) oder Kondensatoren bzw. eine Kombination/Verschaltung aus diesen. Denkbar wäre in einer Ausführungsform, dass eine derartige Verschaltung Mittel zur Wandlung der Energie aufweist, wie z.B. eine Ladeschaltung, die gesteuert durch eine Steuereinheit geringe Batteriespannungen in hohe Therapiespannungen umwandelt. Zusätzlich oder alternativ kann eine solche Verschaltung Schalter/Therapieschalter aufweisen, die gesteuert von der Steuereinheit die Therapiespannung bzw. den Teilbetrag der Therapiespannung entsprechend zuschaltet.

Gemäß einer präferierten Ausführungsform sind die Untereinheiten so miteinander verbunden und synchronisiert, dass der Therapiestrom durch Summation der von den einzelnen Untereinheiten generierten Teilströmen resultiert. Für die Addition der Ströme (z.B. bei Parallelschaltung der Untereinheiten) sind die Untereinheiten über mehr als einen Verbindungsleiter miteinander enthält die Verbindungsleitung mehr als nur einen Leiter.

In der vorliegenden Erfindung ist eine der Untereinheiten als Master, die mindestens eine andere Untereinheit als Slave realisiert bzw. programmiert/konfiguriert. "Master-Rolle" bedeutet in diesem Zusammenhang, dass bei der Detektion eines physiologischen Ereignisses in einem Messsignal, das eine Therapie erforderlich macht, die Untereinheit in der Master-Rolle die Führung über Koordination, Kommunikation, Synchronisation und Therapieabgabe mit den anderen Untereinheiten übernimmt. Dementsprechend ordnen sich die Untereinheiten in der Slave-Rolle der Untereinheit in der Master-Rolle unter, d.h. bei Koordination, Kommunikation, Synchronisation und Therapieabgabe mit den anderen Untereinheiten orientieren sie sich an der Untereinheit in der Master-Rolle.

Vorzugsweise verfügt die als Master arbeitende Untereinheit über eine Detektionseinheit (inkl. Sensingeinheit), die die Notwendigkeit der Therapieabgabe aus der Analyse intrakorporaler Signale ableitet.

Eine erfindungsgemäße Ausführung sieht vor, dass mehrere oder alle Untereinheiten ein elektrisches Messsignal aufnehmen, das physiologische Eigenschaften des Patienten repräsentiert, beispielsweise ein Elektrokardiogramm (EKG). Des Weiteren sind mehrere oder alle Untereinheiten ausgelegt, Ereignisse im elektrischen Messsignal zu detektieren, die eine Therapieabgabe erfordern. In einer Ausführungsform übernimmt dabei jene Untereinheit die Master-Rolle, die als erstes zu einer Detektionsentscheidung kommt. Ferner kann die zum Master gewordene Untereinheit die anderen Untereinheiten in eine Slave-Rolle versetzen.

In einer Ausführungsform der Erfindung umfassen die Untereinheiten jeweils eine Steuereinheit zur Auslösung des Therapiebefehls. Ferner verfügen die Untereinheiten jeweils über eine Kommunikationseinheit u.a. zum Aussenden und Empfang des Therapiebefehls (Die Kommunikationsinformation ist in diesem Fall der Therapiebefehl). Unter Aussenden und Empfang ist auch eine galvanische Kopplung zu verstehen.

Beispielsweise leitet die Untereinheit in der Master-Rolle einen Therapiebefehl sowohl an die eigene Therapieeinheit als auch an die Slave-Untereinheiten weiter.

In einer bevorzugten Ausführungsform ist der Therapiebefehl ausgeführt als ein Signal, in dem der Zeitpunkt der Therapieabgabe, die Polarität der Therapieabgabe und die Dauer, mit der die Teilenergien der einzelnen Untereinheiten zugeschaltet werden, kodiert sind. Damit wird sichergestellt, dass eine synchrone und vorzeichenrichtige Überlagerung der Teilbeiträge der einzelnen Untereinheiten erfolgt.

In einem Ausführungsbeispiel umfassen die Untereinheiten des Weiteren Vorrichtungen zur Zeitmessung mit einer hohen Genauigkeit, z.B. besser als 5 ms. Erkennt die Untereinheit in der Master-Rolle die Notwendigkeit der Therapieabgabe, kann er alternativ zu den anderen gelisteten Methoden die Abgabe der Therapie für eine bestimmte Uhrzeit planen, d.h. alle Untereinheiten schalten zu der Uhrzeit ihren Teilbeitrag synchron frei, oder mit Verzögerung um eine programmierte Dauer relativ zu einem bestimmten Zeitpunkt planen. So ein Zeitpunkt kann z.B. der synchrone Befehl zum Starten des Ladevorganges sein. Die Uhren sind befähigt sich mit externen Uhren (z.B. Funkuhr) zu synchronisieren. Alternativ synchronisieren sich die Uhren nur innerhalb des implantierten Systems. Die Synchronisation der Uhren findet entweder Ereignisgesteuert zum Zeitpunkt der Detektion statt oder regelmäßig in Intervallen dass die Genauigkeit von besser als 5ms gewährleistet wird.

In einer bevorzugten Ausführungsform der Erfindung detektiert eine Untereinheit physiologische Parameter, die eine Therapie erfordern, und gibt daraufhin eine elektrische Spannung gerichtet an den Therapieort ab. Die mindestens eine andere Untereinheit detektiert die Spannungsabgabe der ersten Untereinheit und gibt daraufhin ebenfalls Spannung gezielt auf den Therapieort ab.

Die Untereinheiten verfügen über einen galvanisch an das Gewebe angekoppelten Elektrodenpol der bevorzugt an dem Gehäuse der Untereinheit angebracht ist (Gehäusepol). Dieser ist in einer bevorzugten Ausführungsform das Gehäuse der jeweiligen Untereinheit selbst, das dann elektrisch leitfähig (z.B. metallisch) realisiert ist. Dieser Elektrodenpol ist optional für die Therapieabgabe zuschaltbar. Diese Information kann ebenfalls von dem von der Untereinheit in der Master-Rolle stammenden Therapiebefehl kodiert sein. Dieser Elektrodenpol (Gehäusepol) wird optional auch zum Sensing genutzt.

Alternativ oder zusätzlich sind auf der Verbindungsleitung zwischen den Untereinheiten zusätzliche Elektrodenpole für die Sensingfunktion untergebracht und mit den Sensing/Detektionseinheiten verbunden.

Signale von Sensingelektroden, die nur mit Slave-Untereinheiten verbunden sind, werden von der Sensing/Detektionseinheit der jeweiligen Slave-Untereinheit verarbeitet und das Ergebnis über die Kommunikationseinheit an die Master-Untereinheit übertragen. Zu diesem Zweck sind die Kommunikationstrecken zwischen den Untereinheiten bidirektional.

Gemäß einer Ausführungsform handelt es sich bei der erfindungsgemäßen Anordnung um eine im Körper 120 implantierte oder am Körper applizierte Vorrichtung zur Hochspannungstherapie bestehend aus mindestens zwei Untereinheiten, die energiespeichernde Elemente enthalten, und durch mindestens eine zum Körper hin elektrisch isolierte galvanische Verbindungsleitung miteinander verbunden sind. Dabei sind die Untereinheiten elektrisch so miteinander verbunden, dass die therapeutische Wirkung durch synchronisierte Überlagerung von Teilbeiträgen zur elektrischen Gesamtleistung aus den einzelnen Untereinheiten zustande kommt. Die Synchronisation der Teilbeträge findet über galvanische, elektromagnetische, optische oder mechanisch/akustische Kommunikation zwischen den Untereinheiten statt.

Das System zur Defibrillation des Herzens soll einerseits klein und kostengünstig, andererseits hoch effektiv und sehr zuverlässig sein. Dafür soll es aus Komponenten und Bauteilen aufgebaut sein, die geringere Anforderungen an die Spannungsfestigkeit haben als die im Wirkbereich (im Herzen) erforderliche Spannung. Dieses System soll z.B. auch von außerhalb des Brustkorbes (der Rippen) erfolgreich defibrillieren können, obwohl die Komponenten für Spannungen unter 1000V ausgelegt sind.

### Kurze Beschreibung der Figuren

- Fig. 1a: zeigt ein Beispiel der erfindungsgemäßen Anordnung, welche über zwei Untereinheiten verfügt und an oder im Körper eines Patienten angeordnet ist.
- Fig. 1b: zeigt ein Beispiel der erfindungsgemäßen Anordnung, bei dem die Untereinheiten in einem Gehäuse angeordnet sind.
- Fig. 2a: zeigt ein Beispiel für die Verschaltung der erfindungsgemäßen Untereinheiten
- Fig 2b: zeigt ein Beispiel für die Verschaltung der erfindungsgemäßen Untereinheiten entsprechend der Ausführungsform in Fig. 1b
- Fig 3: zeigt Beispiele einer Überlagerung zweier Spannungen gemäß Ausführungsformen der vorliegenden Erfindung
- Fig. 4: zeigt ein Beispiel für die Verschaltung der erfindungsgemäßen Untereinheiten, wenn die Anordnung drei Untereinheiten aufweist.
- Fig. 5: zeigt ein Beispiel einer bevorzugten geometrischen Formgebung der Untereinheiten.
- Fig. 6 a - j: zeigt beispielhafte Implantationsanordnungen der erfindungsgemäßen Anordnung

### Detaillierte Beschreibung der Figuren

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die

Figuren lediglich typischen Ausgestaltungen der Erfindung wieder und sollen die Erfindung nicht auf die dargestellten Ausgestaltungen beschränken.

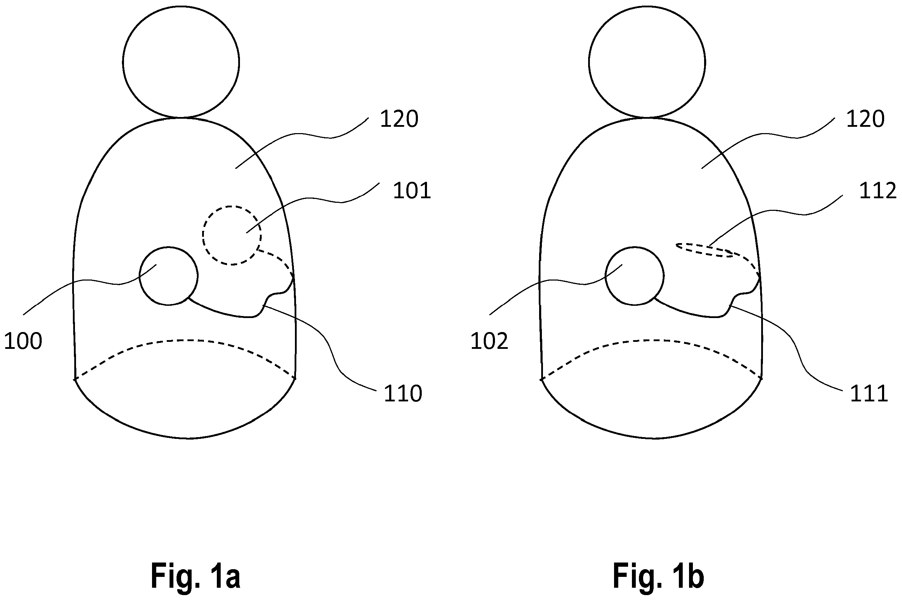

Fig. 1a zeigt das im Körper 120 implantierte System bestehend aus den Untereinheiten 100 und 101 die mit der zum Körper hin elektrisch isolierten galvanischen Verbindungsleitung 110 (Potentialverbinder) miteinander verbunden sind.

Fig 1b zeigt eine alternative Realisierung, bei der die Untereinheiten 100 und 101 in ein gemeinsames Gehäuse (102) gekapselt sind und die Verbindung zu einer entfernt gelegenen Gegenelektrode (112) über eine Zuleitung 111 erfolgt.

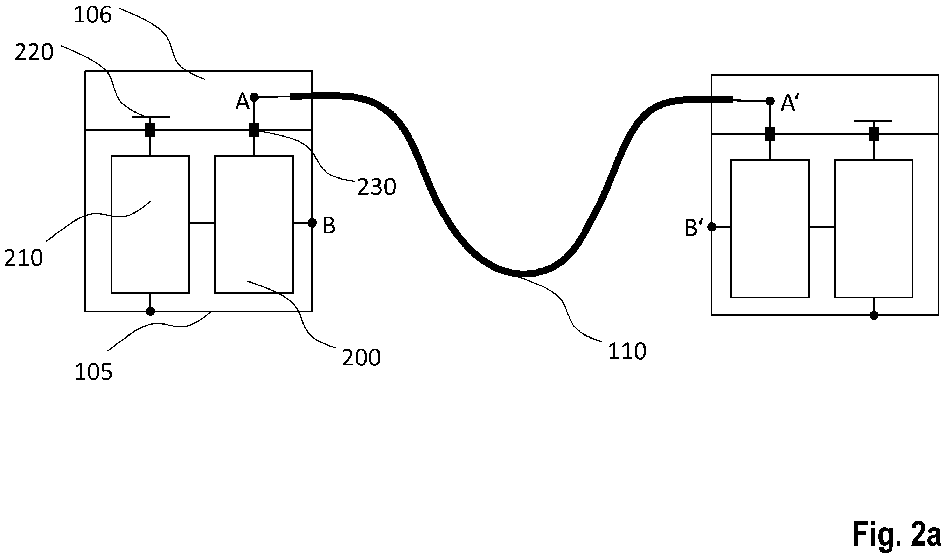

Fig. 2a zeigt die Verschaltung der den Untereinheiten 100 und 101 sowie den prinzipiellen Aufbau einer solcher Untereinheit 100 bestehend aus: dem bevorzugt elektrisch leitend ausgeführten Gehäuse 105 und dem Anschlussbereich (Header) 106, wobei die elektrischen Verbindungen aus dem Inneren von 105 über hermetische Durchführungen 230 in den Anschlussbereich 106 geführt werden. Block 200 fasst repräsentativ die energiespeichernden Elemente zusammen. 210 ist eine Steuereinheit und enthält eine Kommunikationseinheit die über das Sende-/Empfangselement 220 mit anderen Untereinheiten oder externen Geräten Informationen austauscht. Dies ist z.B. eine Antenne für elektromagnetische Kommunikation oder ein Piezoelement für Kommunikation im Ultraschallbereich. Ferner enthält 210 eine Detektionseinheit, über die die Spannung UAB hinsichtlich elektrischer Herzaktivität analysiert wird (Sensing).

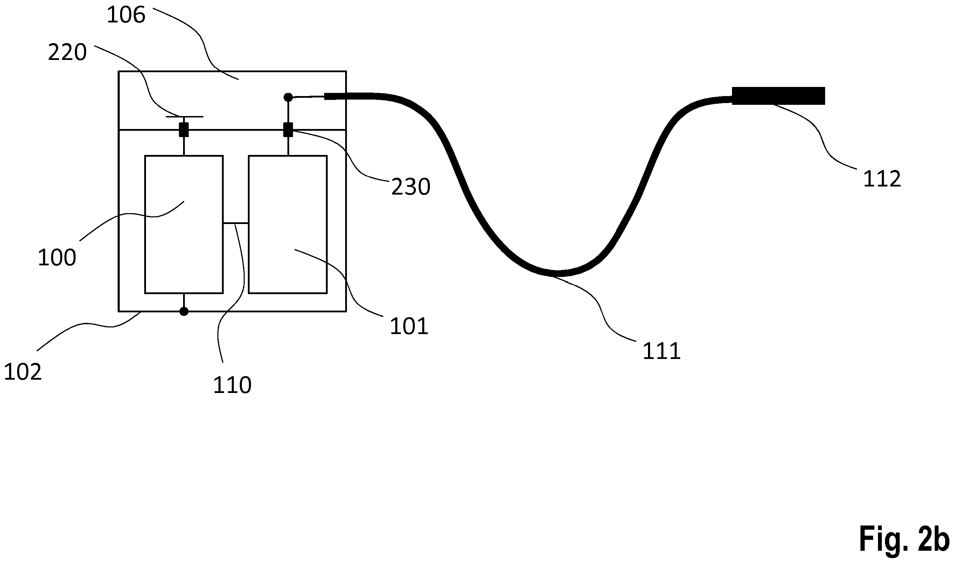

Fig. 2b zeigt den alternativen Aufbau der Lösung gemäß Fig 1b.

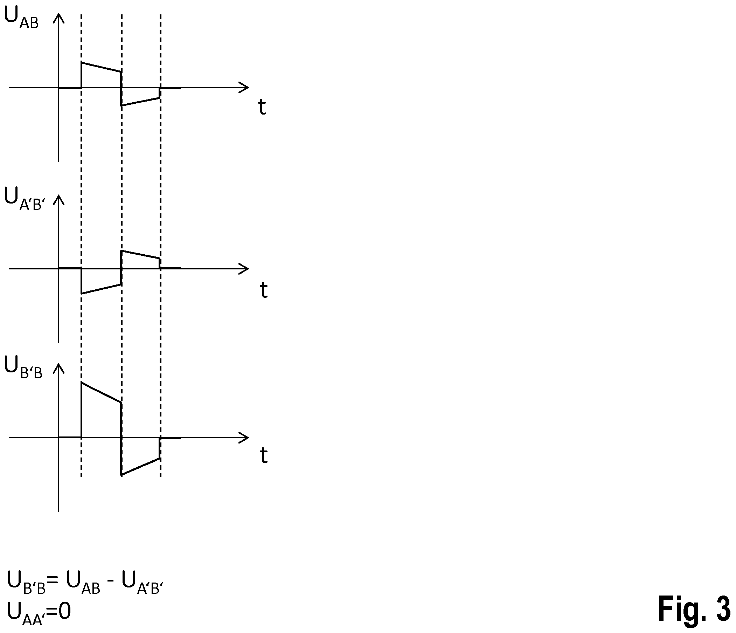

Fig. 3 veranschaulicht das Prinzip der synchronisierten Überlagerung von Teilbeiträgen zur elektrischen Gesamtleistung aus den einzelnen Untereinheiten entsprechend Fig. 2. UAA'=0 wird durch die Verbindungsleitung 110 (Potentialverbinder) sichergestellt.

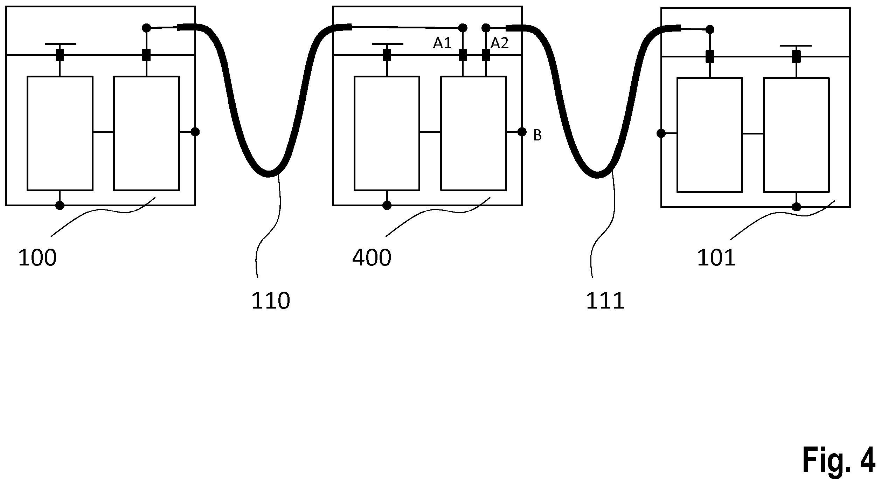

Fig. 4 zeigt exemplarisch eine Verschaltung von 3 Untereinheiten (im Allgemeinen können das mehrere sein). Damit können, je nachdem wie über die Steuereinheiten die elektrisch leitenden Gehäuse (bzw. die Gehäusepole) zugeschaltet sind, verschiedene Therapievektoren eingestellt werden. Die Untereinheit 400 ist prinzipiell wie die Untereinheiten 100 und 101 aufgebaut, nur dass sie die Möglichkeit hat 2 (oder mehrere) Verbindungsleitungen 110 und 111 anzuschließen.

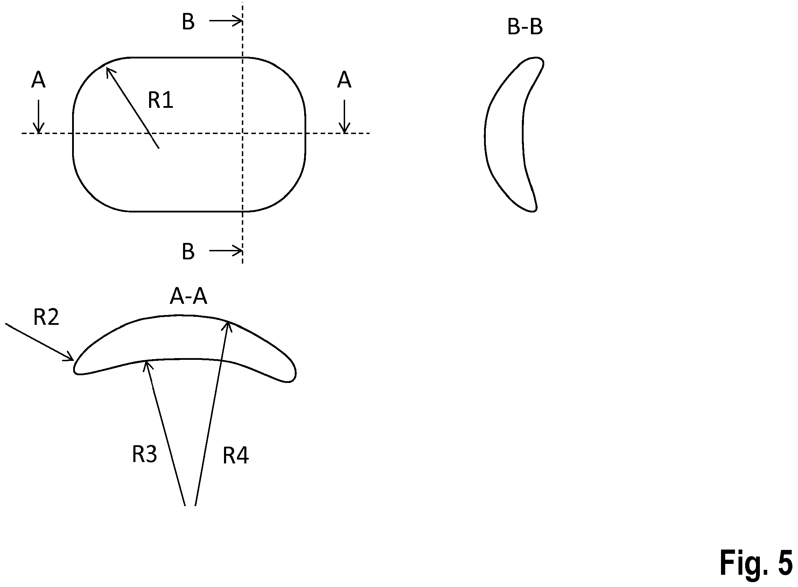

Fig. 5 zeigt eine bevorzugte geometrische Formgebung der Untereinheiten.

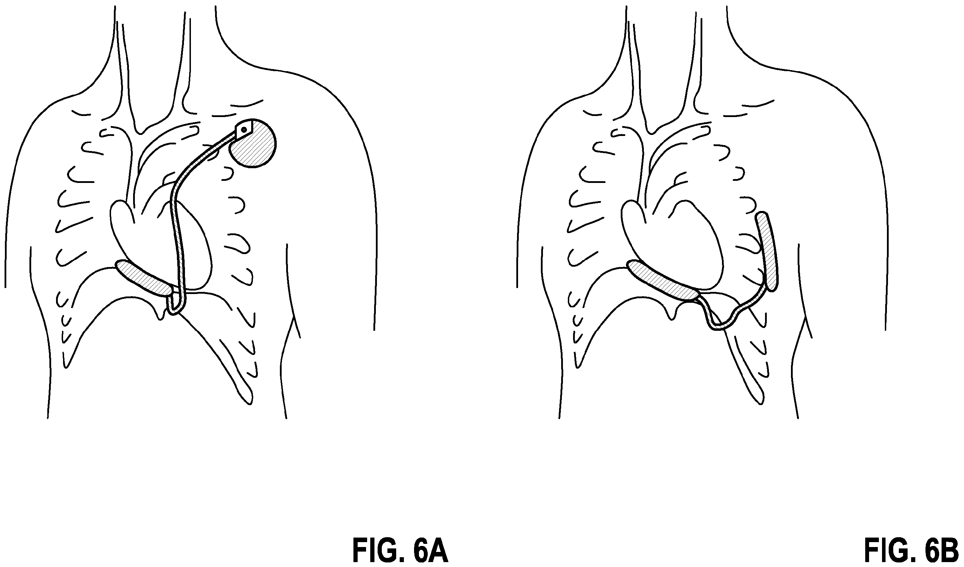

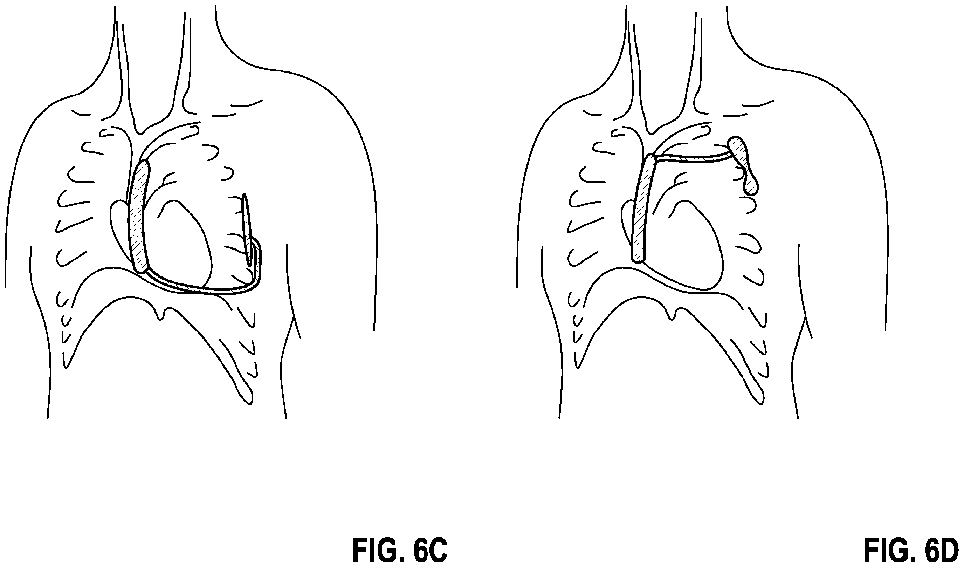

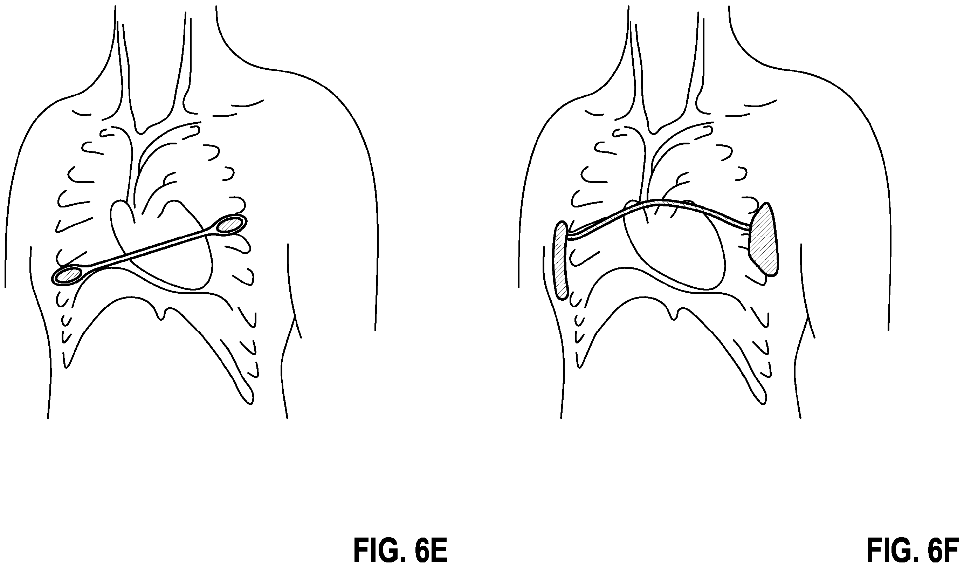

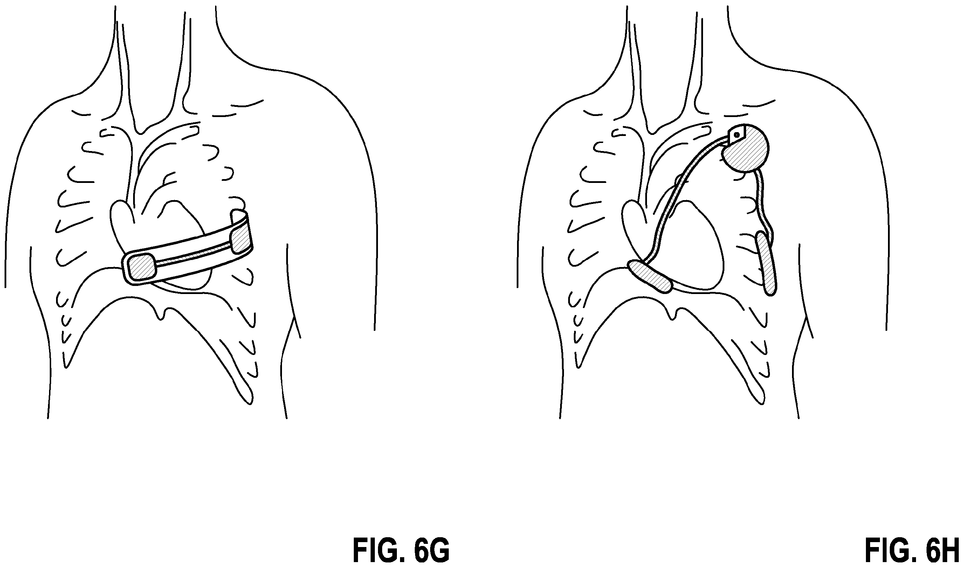

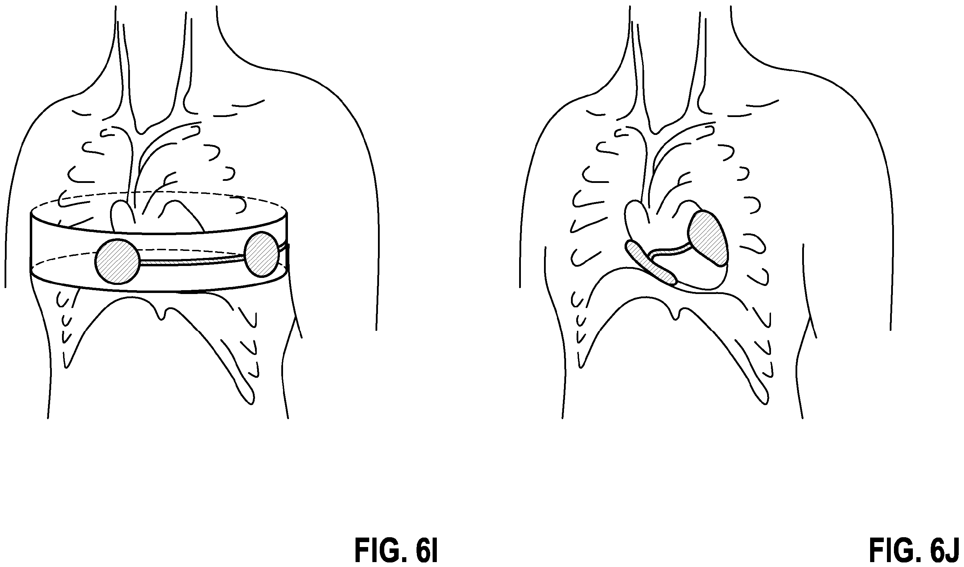

Fig. 6 a bis j zeigen bevorzugte Implantationsanordnungen des Systems.

Die Untereinheiten verfügen über mindestens einen Anschluss, um mindestens eine weitere Untereinheiten über Verbindungsleitungen 110 mit dieser zu verbinden.

Die energiespeichernden Elemente sind Batterien (Primärzellen und/oder Sekundärzellen) und Kondensatoren bzw. eine Kombination aus diesen und sind in Fig. 2 im Block 200 repräsentativ zusammengefasst. Ferner enthält dieser Block 200 Mittel zur Wandlung der Energie, wie z.B. eine Ladeschaltung, die gesteuert durch die Steuereinheit 210 geringe Batteriespannungen in hohe Therapiespannungen umwandelt. Ebenfalls enthält der Block 200 Schalter (Therapieschalter), die gesteuert von der Steuereinheit die Therapiespannung (Teilbetrag) der Anwendung entsprechend zuschaltet. So kann im Beispiel aus Fig. 4 diese Spannung zwischen den Kontaktstellen A1 und A2, A1 und B oder A2 und B durch entsprechende Schalterstellung aufgeschaltet werden.

Die Untereinheiten sind so miteinander verbunden und synchronisiert, dass die Therapiespannung durch Summation der von den einzelnen Untereinheiten generierten Teilspannungen resultiert. Fig. 3 veranschaulicht hierzu ein Beispiel.

Die Untereinheiten sind so miteinander verbunden und synchronisiert, dass der Therapiestrom durch Summation der von den einzelnen Untereinheiten generierten Teilströmen resultiert. Für die Addition der Ströme (z.B. Parallelschaltung der Untereinheit) enthält die Verbindungleitung mehr als nur einen Leiter.

Eine der Untereinheiten ist als Master, die anderen als Slave realisiert bzw. programmiert/konfiguriert Mindestens die als Master arbeitende Untereinheit verfügt über eine Detektionseinheit (inkl. Sensingeinheit), die die Notwendigkeit der Therapieabgabe aus der Analyse intrakorporaler Signale ableitet. Sensingeinheit und Detektionseinheit haben als Teil von der Steuereinheit 210 über deren Verbindung zum Block 200 auch Zugang zur Messspannung UAB (Sensingsignale).

Eine erfindungsgemäße Ausführung sieht vor, dass mehrere oder alle Untereinheiten Detektion ausüben, wobei die erste die zur Detektionsentscheidung kommt die Master-Rolle übernimmt. Die zum Master gewordene Untereinheit versetzt die anderen Untereinheiten in die Slave-Rolle

Die Untereinheiten enthalten jeweils eine Steuereinheit zur Auslösung des Therapiebefehls.

Die Untereinheiten enthalten jeweils eine Kommunikationseinheit u.a. zum Aussenden und Empfang des Therapiebefehls (Die Kommunikationsinformation ist in diesem Fall der Therapiebefehl). Unter Aussenden und Empfang ist auch eine galvanische Kopplung zu verstehen.

Die als Master arbeitende Untereinheit leitet den Therapiebefehl sowohl an die eigene Therapieeinheit (realisiert durch den Block 200) als auch an die Slave-Untereinheiten

Der Therapiebefehl codiert sowohl den Zeitpunkt der Therapieabgabe als auch mit welcher Polarität und für welche Dauern die Teilenergien der einzelnen Untereinheiten zugeschaltet werden. Damit wird sichergestellt, dass eine synchrone und vorzeichenrichtige Überlagerung der Teilbeiträge der einzelnen Untereinheiten erfolgt (vgl. hierzu das Beispiel in Fig. 3). Die Synchronisation erfolgt mit einer Genauigkeit von <5ms.

Die Untereinheiten besitzen Vorrichtungen zur Zeitmessung mit einer Genauigkeit besser 5ms, d.h. Uhren die Kenntnis über die absolute Zeit haben und/oder Zeitintervalle messen können. Erkennt der Master die Notwendigkeit der Therapieabgabe kann er alternativ zu den anderen gelisteten Methoden die Abgabe der Therapie für eine bestimmte Uhrzeit planen (alle Untereinheiten schalten zu der Uhrzeit ihren Teilbeitrag synchron frei) oder mit Verzögerung um eine programmierte Dauer relativ zu einem bestimmten Zeitpunkt planen. So ein Zeitpunkt kann z.B. der synchrone Befehl zum Starten des Ladevorganges sein. Die Uhren sind befähigt sich mit externen Uhren (z.B. Funkuhr) zu synchronisieren. Alternativ synchronisieren sich die Uhren nur innerhalb des implantierten Systems. Die Synchronisation der Uhren findet entweder Ereignisgesteuert zum Zeitpunkt der Detektion statt oder regelmäßig in Intervallen dass die Genauigkeit von besser als 5ms gewährleistet wird.

Die Untereinheiten verfügen über einen galvanisch an das Gewebe angekoppelten Elektrodenpol der bevorzugt an dem Gehäuse der Untereinheit angebracht ist (Gehäusepol). Dieser ist in einer bevorzugten Ausführungsform das Gehäuse der jeweiligen Untereinheit selbst, das dann elektrisch leitfähig (z.B. metallisch) realisiert ist.

Dieser Elektrodenpol ist optional für die Therapieabgabe zuschaltbar. Der Therapiebefehl von der Master-Untereinheit kommend codiert auch diese Information, ob er zugeschaltet werden soll.

Dieser Elektrodenpol (Gehäusepol) wird optional auch zum Sensing genutzt.

Alternativ oder zusätzlich sind auf der Verbindungsleitung zwischen den Untereinheiten zusätzliche Elektrodenpole für die Sensingfunktion untergebracht und mit den Sensing/Detektionseinheiten verbunden.

Signale von Sensingelektroden, die nur mit Slave-Untereinheiten verbunden sind, werden von der Sensing/Detektionseinheit der jeweiligen Slave-Untereinheit verarbeitet und das Ergebnis über die Kommunikationseinheit an die Master-Untereinheit übertragen. Zu diesem Zweck sind die Kommunikationstrecken zwischen den Untereinheiten bidirektional.

Eine Rückmeldung an den Master erfolgt auch hinsichtlich des Fortschrittes des Ladevorgangs damit sichergestellt ist, dass der Befehl zur Therapieabgabe (also synchrone Bereitstellung der Teilbeträge) erst erfolgt wenn alle Untereinheiten den Ladevorgang abgeschlossen haben.

Die Sensing-/Detektionseinheit ist über Schutzschalter vor Überspannungen infolge der Therapieabgabe der eigenen oder anderen Untereinheiten des Systems geschützt. Diese Schalter werden von der Steuereinheit kurz vor Therapieabgabe geöffnet und danach wieder geschlossen. Optional ist die Sensing-/Detektionseinheit zusätzlich über passive Schutzvorkehrungen gegen Überspannungen (auch im Falle externer Defibrillation) geschützt. Hierzu werden in einem Ausführungsbeispiel Schutzdioden eingesetzt.

Damit die an der Therapie beteiligten Elektroden auch zeitnah nach Therapie wieder als Sensing Elektroden dienen können, besitzen die Untereinheiten Vorrichtungen zur Entladung der Elektrodennachpotentiale, z.B. als Kurzschlussschalter ausgeführt.

Die Kommunikation zwischen den Untereinheiten erfolgt in einer der Ausführungen galvanisch. Dieses Kommunikationssignal wird bevorzugt von der Sensingeinheit wahrgenommen und verarbeitet. Für die galvanische Kommunikation sind erfindungsgemäß folgende Lösungen vorgesehen:
Zwischen den Untereinheiten besteht eine bifilare/multifilare Verbindung, d.h. der Verbindungsleiter enthält außer dem Potentialverbinder noch mindestens eine weitere Leitung. Der Stromkreis schließt sich über ein Paar solcher Verbindung. In einer besonderen Ausführung werden zur galvanischen Synchronisation die Pace- und Sensekanäle verwendet wie sei bei konventionellen ICDs z.B. für Atrium und Ventrikel vorliegen.

Der Verbindungsleiter enthält nur den Potentialverbinder. In diesem Fall wird der Stromkreis zur Kommunikation über den Köper geschlossen. Für diese Alternative sieht die Erfindung folgende Realisierungen vor:
In diesen Stromkreis wird ein Wechselstrom eingespeist, der von der Frequenz und Amplitude her nicht stimulierend wirkt (weder für Muskel, Nerven noch Herz). Dabei ist die Frequenz >1kHz und die Amplitude <1mA. Optional sind diese Werte individuell programmierbar. Dieser Wechselstrom ist Träger der Kommunikationsinformation. Die Modulation erfolgt über ein Verfahren das Mittelwertfrei ist (also keine DC Signale durch den Körper gehen). Bevorzugte Modulationsverfahren sind Frequenzmodulation, Pulsweitenmodulation, ggf auch Amplitudenmodulation.

In diesen Stromkreis werden Pulse eingespeist. Die Information wird über die Pulsbreite und Pulsabstand kodiert. Die Pulsbreite liegt bevorzugt unter 10ms (besonders bevorzugt <2ms), der Pulsabstand über 20ms (besonders bevorzugt >80ms). Eine Realisierungsoption verwendet unterschwellige Pulse, die weder Muskel, Nerven noch Herz stimulieren

Eine weitere Realisierungsoption verwendet für Herzmuskel überschwellige Pulse. In dieser Variante sind die Pulse so ausgelegt, dass sie auch therapeutisch, insbesondere als ATP (antitachykarde Stimulation) wirksam sind. Die Synchronisation der Untereinheiten zur Abgabe der Hochspannungstherapie (Teilbeiträge dazu) erfolgt über ein vorab bestimmtes Muster (Pulsbreiten, Pulsabstände die per Design, oder Programmierung festgelegt sind) ausgelöst. Im Falle dass die Mastereinheit während laufender Synchronisatin einen Therapieabbruch entscheidet (ihre Therapieschalter nicht schließt) löst die Slave-Untereinheit zwar dennoch aus, jedoch erreicht dieser Teilbetrag nicht den Körper, da die Untereinheiten in Reihe geschaltet sind (der Stromkreis im Master bleibt offen).

Eine besondere Ausführung sieht vor, dass so ein Puls der Therapiepuls selbst ist. Der Master schaltet seinen Teilbetrag frei, während die Slave-Einheiten die Therapieschalter noch offen haben. Der Stromkreis schließt sich zunächst nur über die Sensingeinheiten der Slaves. Diese nehmen den Therapiepuls des Master wahr und schalten bei Überstreiten einer programmierbaren Amplitudenschwelle auch ihre Teilbeiträge mit einer Reaktionszeit <5ms frei.

Die Kommunikation zwischen den Untereinheiten erfolgt in einer weiteren Ausführung elektromagnetisch:
Dafür sind erfindungsgemäß folgende Lösungen vorgesehen:
Die elektromagnetische Kommunikation bzw. die Radiofrequenzkommunikation ermöglicht den Daten- und Informationsaustausch zwischen den Einheiten (100,101) unabhängig von den galvanischen Verbindungen (110) und mit einer wesentlich höheren Geschwindigkeit und daraus resultierenden kürzeren Reaktionszeiten. Zur elektromagnetischen Kommunikation wird das in den Einheiten standardmäßig enthaltene MICS Band Radiosystem verwendet.

Aus energetischen Gründen wird die elektromagnetische Kommunikation in beiden Einheiten nur im Bedarfsfall aktiviert:
Aktivierung bei jeder Detektion, die zur Therapie bzw. Schockabgabe führt (z.B. während des Ladevorgangs)
Detektionsnachricht der Einheiten untereinander über die bifilare Verbindung (110) Wenn als bifilare Verbindung der ventrikuläre oder atriale Pace/Sense Kanal verwendet wird, wird ein dem Detektionskriterium entsprechendes Signal stimuliert.

Es werden die Fälle "alle Einheiten detektieren", "eine der Einheiten detektiert" abgedeckt, um zu garantieren, dass alle beteiligten Einheiten ihr elektromagnetisches Kommunikationssystem aktivieren.

Die elektromagnetische Kommunikation wird periodisch oder nach Vereinbarung aktiviert, z.B. zum Status oder Datenaustausch, zum Test der Verbindung, etc.

Die jeweils zuerst detektierende Einheit fungiert als Master zur Therapieauslösung via elektromagnetische Kommunikation.

Die Kommunikation zwischen den Untereinheiten erfolgt in einer weiteren Ausführung mechanisch/akustisch, insbesondere im Bereich des Ultraschalls

Als weitere Alternative zur Synchronisation der therapeutischen Teilbeiträge wird erfindungsgemäß auf das durch das Therapiesystem wahrgenommene IEGM/EKG getriggert. In einer bevorzugten Realisierung werden dabei zwecks Störungsminimierung nur eingeschränkte Frequenzbereiche ausgewertet und/oder morphologische Merkmale des IEGM/EKG hinzugezogen.

Das erfindungsgemäße implantierbare System verfügt über Kommunikationsmöglichkeiten zu weiteren implantierten Systemen wie Schrittmachersystemen (insbesondere ILP), Monitoringsystemen (z.B. Looprecordern) und Sensoren (z.B. Blutdrucksensoren) sowie auch zu externen Geräten (z.B. zur Programmierung, Datenübertragung). Die Steuereinheit 210 sendet dabei Therapiebefehle auch an ins Therapiekonzept integrierte Schrittmachersysteme (z.B. postshock pacing oder ATP), oder empfängt Informationen von diesen Geräten zwecks erweiterten Sensings (d.h. zuverlässigere Rhythmuserkennung, Diskriminierung des Tachykardieursprungs, Bewertung der hämodynamischen Relevanz von Rhythmusstörungen anhand von Drucksignalen).

Die geometrische Formgebung der Untereinheiten ist so gestaltet, dass sich diese der physiologischen Formung des Thorax (Rippenkrümmung) bestmöglich anpasst, wenig in der Dicke aufträgt und Druckstellen minimiert. Eine Realisierung ist beispielhaft in Fig. 5 dargestellt. Dazu ist das Gehäuse bevorzugt löffelförmig gestaltet und ist so dimensioniert, dass Längen- und/oder Breitenmaß mindestens 5-mal größer als das Dickenmaß sind. Die Radien der Umlaufkontur sind mindestens R1>5mm. Die Radien der Querschnittkontur sind mindestens R1>1mm. Die Radien der löffelförmigen Wölbung sind mindestens R4>30mm. Dabei ist die konkave Wölbung die durch R3 beschrieben ist, weniger oder höchstens gleich ausgeprägt wie die die durch R4 beschrieben ist. In einer bevorzugten Ausführung ist zusätzlich R3<1000mm.

Die Verbindungsleitung 110 zwischen den Untereinheiten sowie die Leitung 111 sind erfindungsgemäß wie folgt gestaltet:
Die Isolation ist ein biokompatibles Material, bevorzugt Silikon oder Polyurethan oder ein Kombination aus beiden

Die Isolation stellt den Leitungskörper dar und hat mindestens ein Lumen durch das der Potentialverbinder geführt wird.

Der Potentialverbinder besteht aus elektrisch hochleitfähigen Drähten (bevorzugt DFT draht).

Der Potentialverbinder ist zusätzlich isoliert wobei dies Material im Lumen gut gleitend ist.

Der Leitungskörper und hat mehr als ein Lumen, wobei jedes Lumen einen Teil der Drähte des Potentialverbinders führt (Redundanz zur Erhöhung der Zuverlässigkeit). Diese Drähte an den Enden der Leitung an die jeweiligen Stecker zusammengeführt.

Die Lumina im Leitungskörper sind helikoidal verdrillt geführt, um mechanische Spannungen bei Biegung zu minimieren.

Der Leitungskörper besitzt Stützstrukturen zur Verstärkung gegen Quetschbelastung. Diese Strukturen sind als zum Leitungsquerschnitt konzentrische Zwischenschicht ausgeführt wobei die Potentialverbinder innerhalb verlaufen

Die Materialien der Stützstrukturen sind bevorzugt selbst biokompatibel und z.B. Metallfasern, Aramidfasern oder Silikone höherer Shore-härte
Die Stützstrukturen sind als Geflecht oder als über die Länge verteilte Ringe (Struktur wie eine Luftröhre) ausgeführt

Die Implantation der erfindungsgemäßen Vorrichtung erfolgt bevorzugt wie in Fig. 1 a und b sowie Fig. 6. A bis j dargestellt und im Folgenden erläutert.

Fig. 6a : Bauchraum
Gerät 1 befindet sich im Bauchraum.

Das Kabel wird am Xiphoid vorbei über das Sternum zum Gerät 2 getunnelt, das sich an typischer ICD-Stelle unterhalb des Schlüsselbeins befindet.

Fig. 6b : Bauchraum und seitlich am Brustkorb
Das Gerät 1 befindet sich im Bauchraum. Ein Kabel wird neben dem Xiphoid subkutan zum Gerät 2 geführt, das seitlich vom Brustkorb angeordnet ist.

Fig. 6c : Substernale Anordnung
Gerät 1 hat eine lang gestreckte sehr dünne Form und wird unter dem Sternum platziert. Das Kabel wird entweder wie auf dem Bild durch den Interkostalraum zum Gerät 2 getunnelt oder es verlässt den Brustkorb nahe dem Xiphoid, um zum links seitlich am Brustkorb sitzenden Gerät 2 zu gelangen.

Fig. 6d : Sternale Anordnung
Gerät 1 hat eine langgestreckte sehr flache Form und wird auf dem Sternum platziert. Das Kabel wird subkutan außerhalb des Brustkorbs seitlich zum Gerät 2 geführt.

Fig. 6e : Interkostale Anordnung
Die Geräte 1 und 2 sind schmal gestaltet und subkutan aber außerhalb des Brustkorbs mit einem Kabel verbunden. Sie werden zwischen jeweils einem Rippenpaar im Interkostalraum angeordnet. Es ist auch denkbar, dass ein oder beide Geräte breiter sind und sich über mehrere Interkostalspalten erstrecken.

Fig. 6f: Brustkorb
Beide Geräte sitzen subkutan, ggf submuskulär außerhalb des Brustkorbs. Sie sind über ein Kabel, das über das Sternum verläuft miteinander verbunden.

Fig. 6g : Flaches Design mit integrierter Anwendung
Die Geräte 1 und 2 sind sehr flach und ergonomisch gestaltet und in einem flachen Band integriert, das subkutan auf dem Brustkorb implantiert wird.

Fig. 6h : Mögliche Umsetzung mit 3 Geräten
Zwei Geräte sind subkutan aber außerhalb des Brustkorbs und eines im Bauchraum implantiert. Jedes Gerät hat das Potenzial, zu schocken. Der Schockvektor kann variabel eingestellt werden. Eine sinnvolle Anordnung kann sein: vom Bauchraum parallel zu den anderen beiden Geräten zu schocken.

Fig. 6i : Brustgurt
Bei dieser Umsetzung wird das System, bestehend aus mindestens 2 Geräten in einen Gurt oder eine Weste integriert. Diese Form der Umsetzung dient der temporären Nutzung eines Kardiovertersystems.

Fig. 6j : Epikardiale Ausführung
In dieser Ausführung wird ein System, bestehend aus 2 sehr dünnen, ergonomisch vorgebogenen Geräten auf das Epikard gesetzt.

Die erfindungsgemäße Lösung ermöglicht die Realisierung eines S-ICD System basierend auf herkömmlicher kostengünstigerer Implantatstechnologie. Die Funktionalität ist durch eine Softwareanpassung realisierbar, ohne dass neue Hardware entwickelt und gefertigt werden muss. Das zu implantierende Volume ist verteilt und trägt weniger auf als herkömmliche S-ICDs (verbesserter Tragekomfort).

## Patentansprüche

1. Anordnung zur elektrischen Therapie für einen Patienten, wobei die Anordnung dazu ausgelegt ist, zumindest eine elektrische Therapiespannung zu erzeugen, die an einem Therapieort im oder am Körper des Patienten wirkt,
wobei die Anordnung mindestens eine erste und eine zweite Untereinheit umfasst, und jede Untereinheit zumindest einen Energiespeicher aufweist,
wobei jede von einem einzelnen Energiespeicher speicherbare Energiemenge geringer ist als die zur Erzeugung der elektrischen Therapiespannung erforderliche Energiemenge,
wobei jede Untereinheit zumindest eine der folgenden Komponenten aufweist:
- Detektionseinheit zur Detektion von Körperparametern,
- Therapieeinheit zur Erzeugung und/oder Abgabe einer elektrischen Spannung zur elektrischen Therapie,
- Steuereinheit, und/oder
- Kommunikationseinheit,
**dadurch gekennzeichnet, dass**
die Anordnung dazu ausgelegt ist, dass eine Untereinheit bezüglich der Erzeugung und/oder Abgabe einer elektrischen Spannung eine Master-Rolle einnimmt, und die zumindest eine restliche Untereinheit eine Slave-Rolle einnimmt, sodass die zumindest eine sich in der Slave-Rolle befindliche Untereinheit bezüglich Zeitpunkt der Therapieabgabe und/oder Polarität der Therapieabgabe und/oder Dauer der Therapieabgabe an der Untereinheit in der Master-Rolle orientiert.

2. Anordnung nach Anspruch 1, wobei die mindestens erste und zweite Untereinheit jeweils dazu ausgelegt sind, über Energie aus dem Energiespeicher eine elektrische Spannung zu erzeugen, und wobei die Anordnung dazu ausgelegt ist, die elektrische Therapiespannung zu erzeugen, in dem die von der zumindest ersten und zweiten Untereinheit erzeugten Spannungen überlagert werden.

3. Anordnung nach mindestens einem der Ansprüche 1 oder 2, wobei die erste Untereinheit in einem ersten Außengehäuse angeordnet ist, und die zweite Untereinheit in einem zweiten Außengehäuse angeordnet ist.

4. Anordnung nach Anspruch 2, wobei die zumindest erste und zweite Untereinheit in einem gemeinsamen Außengehäuse angeordnet sind.

5. Anordnung nach mindestens einem der Ansprüche 2 bis 4, wobei die von den zumindest zwei Untereinheiten erzeugten Spannungen so überlagert werden, sodass die Amplitude der überlagerten Spannung höher ist als die Amplitude einer durch eine Untereinheit erzeugten Spannung.

6. Anordnung nach mindestens einem der vorigen Ansprüche, wobei die von den zumindest zwei Untereinheiten erzeugten Spannungen so überlagert werden, dass die Spannungen am Therapieort im Wesentlichen phasensynchron sind.

7. Anordnung nach mindestens einem der vorigen Ansprüche, wobei jede Untereinheit dazu ausgelegt ist, zumindest mit einer anderen Untereinheit zu kommunizieren, wobei die Kommunikation galvanisch, elektromagnetisch, optisch, mechanisch und/oder akustisch stattfindet.

8. Anordnung nach mindestens einem der vorigen Ansprüche, wobei mindestens eine Untereinheit eine Steuereinheit aufweist, wobei die Steuereinheit dazu ausgeführt ist, einen Therapiebefehl auszugeben, wobei der Therapiebefehl Informationen über zumindest einen der folgenden Parameter trägt:
- Angaben zu Phase der jeweiligen von der Untereinheit bereitgestellten Spannung,
- Zeitpunkt der Therapieabgabe,
- Polarität der Therapieabgabe, und/oder
- Dauer der Therapieabgabe bezogen auf die jeweilige Untereinheit.

9. Anordnung nach mindestens einem der vorigen Ansprüche, wobei jede Untereinheit zumindest eine Detektionseinheit zur Detektion von Körperparametern aufweist und wobei jene Untereinheit eine Master-Rolle erhält, die einen therapieerfordernden Körperparameter zuerst, d.h. vor allen anderen Untereinheiten, detektiert.

10. Anordnung nach mindestens einem der vorigen Ansprüche, wobei die Anordnung mindestens eine Verbindungsanordnung aufweist, wobei die mindestens zwei Untereinheiten durch die Verbindungsanordnung elektrisch verbunden sind, und wobei die Verbindungsanordnung vom Körper des Patienten elektrisch isoliert ist.

11. Anordnung nach Anspruch 10, wobei die mindestens zwei Untereinheiten über die Verbindungsanordnung seriell, antiseriell, parallel oder sternförmig verbunden sind.

12. Anordnung nach Anspruch 10, wobei die Anordnung dazu ausgelegt ist, über die mindestens eine Verbindungsanordnung eine wesentliche Phasensynchronität der von den mindestens zwei Untereinheiten erzeugten Spannungen am Therapieort zu erzeugen.

13. Anordnung nach zumindest einem der vorigen Ansprüche, wobei die zumindest zwei Untereinheiten
- jeweils eine Batterie umfasst, und/oder
- jeweils einen Kondensator umfasst, und/oder
- jeweils einen subkutanen implantierbaren Kardioverter-Defibrillator (sICD) darstellt.

## Claims

1. Arrangement for electrical therapy for a patient, the arrangement being configured to produce at least one electrical therapeutic voltage, which acts at a therapy site in or on a patient's body,
the arrangement comprising at least a first and a second subunit,
and each subunit having at least one energy store,
each amount of energy storable by an individual energy store being smaller than the amount of energy required to produce the electrical therapeutic voltage,
each subunit having at least one of the following components:
- a detection unit to detect body parameters,
- a therapy unit to produce and/or output an electrical voltage for electrical therapy,
- a control unit, and/or
- a communication unit,
**characterised in that**
the arrangement is designed such that one subunit assumes a master role with respect to producing and/or outputting an electrical voltage, and the at least one other subunit assumes a slave role, so that the at least one subunit playing the slave role orients itself with respect to a point in time and/or polarity and/or duration of the therapy output at the subunit playing the master role.

2. Arrangement according to claim 1, wherein the at least first and second subunits are each configured to produce an electrical voltage by means of energy from the energy store, and wherein the arrangement is configured to produce the electrical therapeutic voltage by superimposing the voltages produced by the at least first and second subunits.

3. Arrangement according to at least one of claims 1 or 2, wherein the first subunit is arranged in a first outer housing, and the second subunit is arranged in a second outer housing.

4. Arrangement according to claim 2, wherein the at least first and second subunits are arranged in a common outer housing.

5. Arrangement according to at least one of claims 2 to 4, wherein the voltages produced by the at least two subunits are superimposed so that the amplitude of the superimposed voltage is higher than the amplitude of a voltage produced by a subunit.

6. Arrangement according to at least one of the preceding claims, wherein the voltages produced by the at least two subunits are superimposed so that the voltages are substantially in-phase at the therapy site.

7. Arrangement according to at least one of the preceding claims, wherein each subunit is configured to communicate at least with one other subunit, the communication being implemented galvanically, electromagnetically, optically, mechanically, and/or acoustically.

8. Arrangement according to at least one of the preceding claims, wherein at least one subunit has a control unit, wherein the control unit is configured to output a therapy command, wherein the therapy command carries information about at least one of the following parameters:
- details relating to a phase of the particular voltage provided by the subunit,
- a point in time that the therapy is output,
- a polarity of the therapy that is output, and/or
- a duration of the therapy that is output based on the particular subunit.

9. Arrangement according to at least one of the preceding claims, wherein each subunit has at least one detection unit for detecting body parameters, and wherein the subunit that is the first to detect the body parameter calling for therapy, i.e. before all other subunits, assumes a master role.

10. Arrangement according to at least one of the preceding claims, wherein the arrangement has at least one connection arrangement, wherein the at least two subunits are electrically connected by the connection arrangement, and wherein the connection arrangement is electrically insulated from the patient's body.

11. Arrangement according to claim 10, wherein the at least two subunits are connected in series, in anti-series, in parallel, or in a star shape via the connection arrangement.

12. Arrangement according to claim 10, wherein the arrangement is configured to produce at the therapy site a substantial phase synchronicity of the voltages produced by the at least two subunits via the at least one connection arrangement.

13. Arrangement according to at least one of the preceding claims, wherein the at least two subunits
- comprise a battery each, and/or
- comprise a capacitor each, and/or
- each represent a subcutaneously implantable cardioverter-defibrillator (sICD).

## Revendications

1. Ensemble de thérapie électrique pour un patient, où l'ensemble est prévu pour générer au moins une tension thérapeutique électrique qui agit au niveau d'un emplacement de thérapie dans ou sur le corps du patient,
où l'ensemble comprend au moins une première et une deuxième sous-unité, et chaque sous-unité présente au moins un accumulateur d'énergie,
où chaque quantité d'énergie pouvant être stockée par un accumulateur d'énergie individuel est inférieure à la quantité d'énergie nécessaire pour la génération de la tension thérapeutique électrique,
où chaque sous-unité présente au moins un des composants suivants :
- une unité de détection pour la détection de paramètres corporels,
- une unité de thérapie pour la génération et/ou la délivrance d'une tension électrique pour la thérapie électrique,
- une unité de commande, et/ou
- une unité de communication,
**caractérisé en ce que**
l'ensemble est prévu de sorte qu'une sous-unité prend un rôle de maître en ce qui concerne la génération et/ou la délivrance d'une tension électrique, et l'au moins une sous-unité restante prend un rôle d'esclave, de sorte que l'au moins une sous-unité se trouvant dans le rôle d'esclave oriente en ce qui concerne le moment de la délivrance de la thérapie, et/ou la polarité de la délivrance de la thérapie, et/ou la durée de la délivrance de la thérapie, au niveau de la sous-unité dans le rôle de maître.

2. Ensemble selon la revendication 1, dans lequel les au moins une première et une deuxième sous-unité sont prévues pour générer une tension électrique par l'énergie provenant de l'accumulateur d'énergie, et où l'ensemble est prévu pour générer la tension thérapeutique électrique en ce que les tensions générées par l'au moins une première et une deuxième sous-unité sont superposées.

3. Ensemble selon au moins une des revendications 1 ou 2, dans lequel la première sous-unité est disposée dans un premier boîtier extérieur, et la deuxième sous-unité est disposée dans un deuxième boîtier extérieur.

4. Ensemble selon la revendication 2, dans lequel les au moins une première et une deuxième sous-unité sont disposées dans un boîtier extérieur commun.

5. Ensemble selon au moins une des revendications 2 à 4, dans lequel les tensions générées par les au moins deux sous-unités sont superposées de sorte que l'amplitude de la tension superposée est supérieure à l'amplitude d'une tension générée par une sous-unité.

6. Ensemble selon au moins une des revendications précédentes, dans lequel les tensions générées par les au moins deux sous-unités sont superposées de sorte que les tensions sont essentiellement synchronisées en phases au niveau de l'emplacement de thérapie.

7. Ensemble selon au moins une des revendications précédentes, dans lequel chaque sous-unité est prévue pour communiquer au moins avec une autre sous-unité, où la communication a lieu de manière galvanique, électromagnétique, optique, mécanique et/ou acoustique.

8. Ensemble selon au moins une des revendications précédentes, dans lequel au moins une sous-unité présente une unité de commande, où l'unité de commande est conçue pour délivrer une commande de thérapie, où la commande de thérapie porte des informations concernant au moins un des paramètres suivants :
- des indications concernant la phase de la tension respective fournie par la sous-unité,
- le moment de la délivrance de la thérapie,
- la polarité de la délivrance de la thérapie, et/ou
- la durée de la délivrance de la thérapie sur la sous-unité respective.

9. Ensemble selon au moins une des revendications précédentes, dans lequel chaque sous-unité présente au moins une unité de détection pour la détection de paramètres corporels et dans lequel une sous-unité prend un rôle de maître qui détecte un paramètre corporel nécessitant une thérapie en premier lieu, c'est-à-dire avant toutes les autres sous-unités.

10. Ensemble selon au moins une des revendications précédentes, où l'ensemble présente au moins un ensemble de liaison, où les au moins deux sous-unités sont reliées électriquement par l'ensemble de liaison et où l'ensemble de liaison est isolé électriquement du corps du patient.

11. Ensemble selon la revendication 10, dans lequel les au moins deux sous-unités sont reliées en série, en anti série, en parallèle ou en forme d'étoile par le biais de l'ensemble de liaison.

12. Ensemble selon la revendication 10, où l'ensemble est conçu pour générer une synchronisation de phase essentielle des tensions générées par les au moins deux sous-unités sur l'emplacement de thérapie par le biais de l'au moins un ensemble de liaison.

13. Ensemble selon au moins une des revendications précédentes, dans lequel les au moins deux sous-unités
- comprennent respectivement une batterie, et/ou
- comprennent respectivement un condensateur, et/ou
- représentent respectivement un défibrillateur-cardioverteur implantable en sous cutané (sICD).
